# EUROPEAN PATENT APPLICATION

(11) **EP 2 209 076 A1**
(43) Date of publication of application: **21.07.2010**
(21) Application number: 09000334.4
(22) Date of filing: 13.01.2009
(51) Int. Cl.: G06F 19/00, A61M 5/142

(54) **Ambulatory infusion device with plunger position memory**

(71) Applicant: F.HOFFMANN-LA ROCHE AG, 4070 Basel (CH); Roche Diagnostics GmbH, 68305 Mannheim (DE)
(72) Inventor: Remde, Axel, 3432 Lützelflüh-Goldbach (CH)
(74) Representative: Poredda, Andreas

(57) **Abstract**

The invention is directed towards an ambulatory infusion device for the infusion of a liquid drug into a patient's body over an extended time period,
characterized in that the infusion device comprises
a position memory (85), configured to store at least one stored loading position of the plunger, and in that
the controller (80) is configured to retrieve a stored loading position from the position memory (85) and to control the drive (70) to displace the plunger into the retrieved loading position.
The invention is further directed towards a system for ambulatory drug infusion comprising such an ambulatory infusion device as well as a filling apparatus and towards a method for loading a cartridge into such a device.

## Description

The present invention is related to ambulatory infusion devices for the infusion of a liquid drug into a patient's body over an extended time period, to methods for loading a cartridge into such ambulatory infusion devices as well as to systems for ambulatory drug infusion, comprising an infusion device and a filling apparatus.

Ambulatory infusion devices for the infusion of a liquid drug over an extended time period are known in the art for a number of therapies. In particular, such devices form the basis for a state-of-the-art therapy of Diabetes Mellitus by CSII (Continuous Subcutaneous Insulin Infusion). Such an ambulatory infusion device is disclosed, for example, in the WO 2003053498 A2 to which reference is made for the general design and features of such devices according to the state of the art. In the following, an ambulatory infusion device according to the technical field as stated above and in particular an ambulatory infusion device in accordance with the present invention is referred to as "device".

Besides diabetes therapy, those devices may be used for a number of further therapies, such as cancer treatment or pain therapy, without requiring substantial modification. The following description of the present invention mainly refers to CSII therapy without limiting the invention to this specific application.

While present devices are comparably comfortable and easy to use during application, some service operations, such as replacing the catheter with the subcutaneous cannula and replacing the drug cartridge are to be performed repeatedly every few days by the user. It is therefore desired to keep these steps as simple, fail-proof and quick as possible without increasing the overall therapy costs.

In the following, a person operating a device according to the technical field is referred to as the "user" of the device. The user is typically the patient carrying the device for therapeutic purposes, but may also be a parent, a partner, a nurse, or the like.

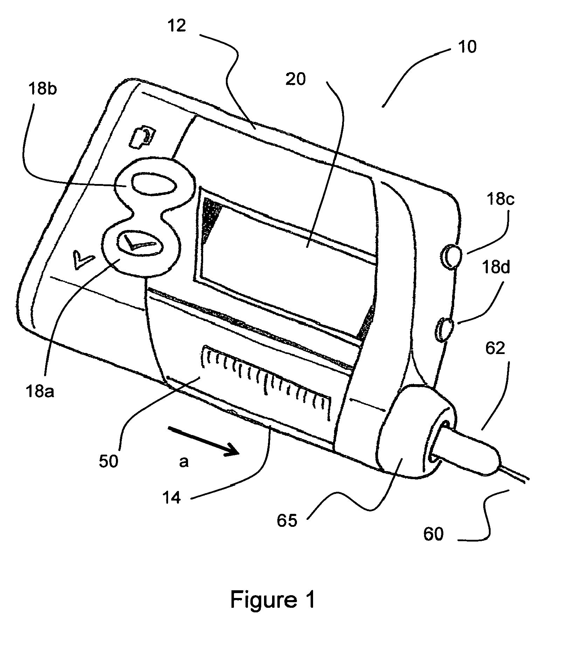

Figure 1 shows an outside view of an exemplary infusion device 10 which may be a sate-of-the-art-device as well as a device in accordance with the present invention. The infusion device 10 comprises a cartridge compartment 14 with a transparent window. The cartridge compartment 14 is designed to receive a cylindrical cartridge 50 of, for example, 3 ml or 300 I.U. (International Units) of insulin as maximum filling volume. The cartridge compartment 14 is closed by a removable adapter 65 which is in screwed and water-tight engagement with the housing 12. The cartridge 50 is fluidically connected with a catheter 60 via a catheter connector 62, wherein the connection is secured by the adapter 65. For the drug administration, a plug (not visible in Figure 1) is arranged inside the cartridge body and is controllably displaced in the delivery direction *a*, thus forcing drug out of the cartridge. The displacement of the plug is performed by a plunger (not visible in Figure 1) which is in engagement with the plug. The plunger is comprised by a drive of the infusion device with the displacement being controlled by an electronic controller. Exemplary drives are disclosed, among others, in the documents US6248093B1 or EP0991440B1 .The cartridge 50 is axially supported against the delivery direction *a* by the adapter 65.

For loading a drug cartridge 50 which is filled to its maximum filling volume into a device as shown in Figure 1, the following steps must be performed by the user, starting from an unloaded pump without adapter 65 and the plunger of drive being in a fully retracted position inside the cartridge compartment 14:

The filled cartridge 50 is hold such that its catheter connector points upwards, e.g., by placing it standing on a table. The adapter 50 is then coupled with the cartridge 50 by putting it over the catheter-connector of the cartridge 50 with a slight pushing-force, thus establishing a snap-fit engagement of the cartridge 50 and the adapter 65. Next, the catheter connector 62 of a fresh catheter 60 is secured to the adapter 65 by a screwing motion. Then, the cartridge 50 is inserted into the cartridge compartment 14. By finally rotating the adapter 65, the adapter is coupled with the housing 12 in a screwing manner, simultaneously displacing the cartridge 50 into its final axial position and engaging the plug of the cartridge 50 with the plunger. For replacing the drug cartridge, the user removes the old cartridge 50 along with the adapter 65 by rotating the adapter 65, thus releasing the screwed engagement of the adapter 65 and the housing 12, followed by inserting the replacement cartridge into the cartridge compartment as described above. Between removing the old cartridge and loading the replacement cartridge into the device, the plunger is fully retracted via a corresponding user command entered via the user interface 18.

For a cartridge filled to its maximum filling volume, the plug of the cartridge is in a most proximal position with its proximal front face being substantially flush with the open proximal end of the cartridge body, such that the inner volume of the cartridge as defined by the cartridge body and the plug is maximal.

Here and in the following, the terms "proximal" and "distal" are referred to with respect to the delivery direction *a*, wherein the arrow indicating the delivery direction *a* points from proximal to distal.

The cartridge is, however, not always filled to this maximum filling volume when being inserted into the device for several reasons. First, there is typically some variability associated with the manual filling process of the cartridge. Second, stability of drugs such as insulin formulations is given in a typical plastic cartridge only for a limited time of some days. This time decreases with increasing ambient temperature. Therefore, patients having a low drug-demand, such as children on CSII therapy, as well as patients living in warm climates, tend to fill the cartridges not to the maximum filling volume, but only to two thirds, one half, or even less. Third, cartridges which have only been partly emptied are sometimes temporary removed and again re-inserted later on. This is the case, for example, if a patient goes on a weekend trip during which he does not want to be bothered with cartridge replacement. In such situations, the only partly emptied cartridge is replaced by a fresh one before the trip. The removed cartridge is re-inserted again later on.

For some state-of-the-art devices, the infusion device may automatically detect the plug position within the cartridge body and establish correct engagement with the plunger by slowly displacing the plunger from its fully retracted position into the delivery detection until the infusion device detects that the plunger touches the plug. Here, the engagement between the plunger and the plug is a pure pushing engagement for pushing the plug into the delivery direction.

For some other devices, automatically establishing the engagement between the plunger and the plug is not possible. This is the case where the cartridge plug comprises an inner-threaded hole which engages an outer-threaded end section of the plunger for secure engagement in both pushing and pulling direction. The same holds true for some other types of engagement, for example using a bayonet connector.

The plunger position for correct engagement of the cartridge plug is in the following referred to as "loading position". The overall process of putting a cartridge into the device and in particular of replacing a cartridge is referred to as "loading". The term "inserting" may additionally be used for clarity reasons and referrers to the physical insertion.

For devices not allowing automatic detection of the plug position within the cartridge body, the plunger has to be manually displaced into the correct loading position by the user prior to inserting the cartridge into the cartridge compartment in case the cartridge to be loaded into the device is not filled to its maximum filling volume. The term 'manual' is used in this context to indicate that a manual user action is required, while the actual displacement is performed by operating the drive via the device controller.

As described in the following, the step of manually displacing the plunger into the loading position is somewhat tricky and mistakes are known to occur if the user is visually and/or physically handicapped. Diabetes is often accompanied by such handicaps.

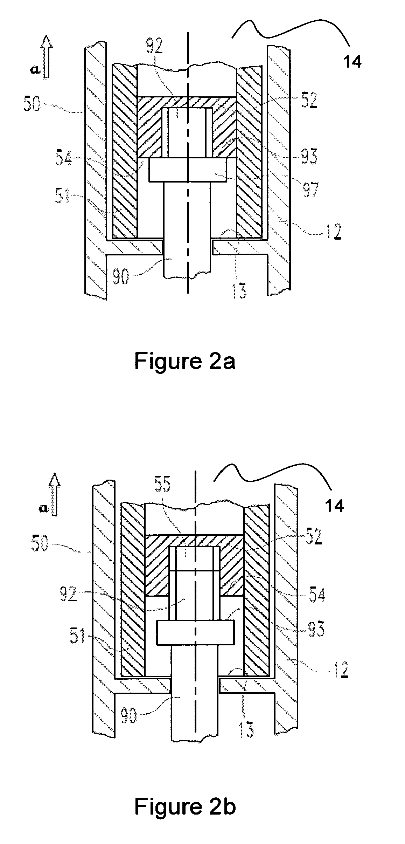

Reference is now made to Figure 1 and Figure 2. Figure 2a and Figure 2b schematically show the ground section of a cartridge compartment 14 with the cartridge 50 and the plunger 90 in threaded engagement with a cartridge plug 52. For manually displacing the plunger 90 into the loading position, the cartridge 50 with the already attached adapter 50 and the catheter 60 is hold outside the housing 12 next to the transparent window of the cartridge compartment 14 in an axial position which corresponds to the final position of the cartridge 50 when correctly placed in the cartridge compartment 14. Using the input unit 18, for example the buttons 18c and 18d, the plunger 90 can manually be displaced in proximal and distal direction into the correct loading position in which the distal end face 93 of the connector plate 91 is in alignment with the proximal front surface 54 of the plug 52. While displacing the plunger 90, the plunger position is checked against the plug position by the user via the transparent window of the cartridge compartment. In state-of-the-art devices, this procedure has to be performed each time a cartridge is loaded into the device if the cartridge is not filled to its maximum filling volume.

In the following, a loading position into which the plunger is displaced by manually adjusting its position via the user interface 18 is referred to as "manual loading position". After displacing the plunger 90 into the loading position, the cartridge 50 is loaded into the cartridge compartment 14 as described above. Simultaneously with twisting the adapter 65, the cartridge 50 is twisted via the engagement of the cartridge 50 and the adapter 65, thus screwing the threaded bold 92 into a corresponding threaded hole in the plug 52.

While Figure 2a shows a situation where the plunger 90 was displaced into the correct loading position prior to inserting the cartridge into the cartridge compartment 14, Figure 2b shows a situation where the projecting length of the plunger 90 into the cartridge compartment 14 is too small. Here, the threaded bold 92 does not engage the threaded hole of the plug 52 on its full length, such that a cavity 55 remains in the plug 52. In this case, the engagement between the threaded bold 92 and the plug 52 is not secure, a play remains between the threaded bold 92 and the plug 52, and the plug 52 may deform and bend into the cavity 55 under the influence of the fluidic drug pressure, thus reducing the mechanical stiffness. All of these effects are undesired. If the plunger 90 is positioned such that the projecting length is larger than required for correct engagement, drug is forced out of the cartridge 50 in an uncontrolled way when loading it into the cartridge compartment 14.

It is the objective of the present invention to simplify the process of loading a drug cartridge into a device of the above-described type and in particular of displacing the plunger into the loading position. The problem is solved based on the insight that the loading position when inserting a new cartridge often corresponds to the loading position of another previously inserted cartridge or another well-defined previous plunger position. It is therefore concluded that the device may be improved by storing a plunger position in a position memory and subsequently recalling it as loading position where required. This allows largely reducing or even avoiding the necessity for manually displacing the plunger into the loading position.

According to a first aspect of the invention, ambulatory infusion devices with the following features are provided:
a) a housing having a cartridge compartment, the cartridge compartment being designed to receive a cartridge;
b) a drive having a plunger, the plunger being adapted to engage a plug of the cartridge, the drive being configured to displace, via the plunger, the plug within cartridge body in a controlled way;
c) an electronic controller, configured to control operation of the drive,
d) a position memory, configured to store at least one loading position of the plunger, wherein
e) the controller is configured to retrieve a stored loading position from the position memory and to control the drive to displace the plunger into the retrieved loading position.

The position memory of an infusion device in accordance with the invention is preferably a RAM or writable ROM memory and may be integral with further data memory and/or the controller of the device. A loading position which may be stored in the position memory is generally an arbitrary plunger position rather than a reference position defined by the device design.

By providing such a device, the need for entering a manual loading position is largely reduced. It has to be performed by the user only if the loading position is different from a loading position which is stored in the position memory. If a user, for example, generally fills the cartridge only to one half of the maximum filling volume, he has to manually displace the plunger into the loading position only the first time he does so, followed by storing this position. Subsequently, this loading position may always be retrieved from the position memory.

A loading position which is stored in the position memory is in the following referred to as "stored loading position". A stored loading position which is retrieved from the position memory is referred to as "retrieved loading position". If a retrieved loading position or a fixed reference position of the plunger is manually modified, it becomes a manual loading position as defined above.

A device according to the invention is especially favorable if the plunger and the plug of the cartridge couple by a releasable threaded engagement or by another structure not allowing automatic detection of the plug position and automatic establishing of the engagement. For those devices, automatic detection of the plug position in the cartridge and automatically coupling the plug and the plunger is not possible as described above. For alternative devices allowing automatic detection of the plug position in the cartridge body and automatic displacement of the plunger into the loading position, the invention is advantageous since it avoids the time-consuming process of fully retracting the plunger and subsequently displacing it into the delivery direction till it touches the plug each time a cartridge is loaded into the device.

The device may be configured for the entry of a manual loading position via an input unit and may be further configured to store the manual loading position as stored loading position in the position memory. The controller advantageously controls the drive to displace the plunger while entering the manual loading position such that the actual plunger position may be checked by the user during entry. This may be achieved by displacing the plunger via two buttons as described above in proximal and/or distal direction. While displacing the plunger in to the loading position, the corresponding filling volume is advantageously indicated via a device display. Alternatively, the device may allow the user to enter a manual loading position, or a desired filling volume via an input unit, followed by displacing the plunger into the manual loading position. The device may further be configured to receive a loading position via a communication interface, for example from a remote controller or a PC running configuration software. Furthermore, loading positions may be stored into the position memory by the device manufacturer as typically used loading positions.

Besides loading positions retrieved from the position memory and/or manual loading postions, the plunger may advantageously be displaced into a fixed reference position. The reference position may be a fully retracted position of the plunger, thus being the loading position for a cartridge filled to its maximum filling volume. This is especially favorable for patients using fully as well as partly filled cartridges. However, the reference position may be any other plunger position that may be assumed by the plunger in a repeatable way.

After removing a cartridge, the controller of the device may control the drive to directly displace the plunger into a retrieved loading position stored in the position memory or a manual loading position. Alternatively, it may control the drive to generally displace the plunger first into the reference position, followed by optinally displacing it into a loading position retrieved from the position memory or a manual loading position. For several known designs of the drive, for example drives in accordance with US6248093B1 or EP0991440B1 the fully retracted reference position is the only absolute plunger position which may be detected, while controlling the plunger to assume any other position is controlled relative to the reference positions. Therefore, generally displacing the plunger into the reference position prior to displacing it into the loading position may be advantageous for safety and reproducibility reasons. For alternative designs which allow absolute measurement of the plunger position, e.g., via a Position Sensing Device, PSD, this may not be required.

In some embodiments, the position memory comprises a set of memory registers each of which can store a loading position which may subsequently be retrieved. This is advantageously, for example, if the patient uses drug cartridges filled to a set of different filling volumes which may be different, for example, in summer time and winter time. Besides memory, there is no general limit for the number of memory registers. For practical purposes, it may be in the range of three to five. The individual loading positions of the set of loading positions are advantageously identified by identifiers such as characters or numbers, by the cartridge filling volume corresponding to the plunger position, and/or further useful information such as the storage date. The information may be provided on a device display.

If a limited set of memory registers is provided and a new loading position is to be stored, it is generally necessary to replace another stored loading position. If the memory registers are identified by identifiers such as numbers or characters, the user may easily select the stored loading position to be replaced. Alternatively or additionally, the position memory may store the date of storing a loading position along with the loading position and select the oldest one to be replaced. Alternatively, the memory register storing the loading position with the minimum difference to the loading position to be stored may be selected for replacement.

In some embodiments, the controller is configured modify a retrieved loading position based input entered via an input unit. Manually modifying a retrieved loading position prior to loading a cartridge into the device may be required in order to compensate for variations of the filling volume which are typical for manual filling processes. The controller is advantageously configured to control the drive to displace the plunger while modifying the loading position but may also be configured to displace the plunger subsequent to entering the modification. The modification is advantageously entered as modification of the filling volume rather than the plunger position.

The device may be configured to compare a manual loading position and a stored loading position and to selectively replace the stored loading position in the position memory by the manual loading position in dependence of the comparison result. The comparison is advantageously carried out by the device controller. This type of embodiment enables the device to automatically detect if a loading position should be stored into the position memory. If a manual loading position only deviates from a stored loading position by a small amount, it may be assumed that the deviation results from an adjustment for compensating the variability in the filling process. In this case, the manual loading position should not be stored into the position memory. If the manual loading position, in contrast, substantially deviates from a stored loading position, it may be assumed the filling volume of the cartridge is substantially different from previous filling volumes and should be stored in the position memory. Therefore, the device may store a filling deviation threshold and may be configured to store a manual loading position only if it deviates for more than the filling deviation threshold. For manually filling a state-of-the-art-cartridge of 300 I.U. maximum filling volume, this maximum filling deviation, may, for example, be in the range of 3 I.U. to 8 I.U.

If a manual loading position is entered by modifying a retrieved loading position as described above, the manual loading position may be compared only with this retrieved loading position. In this case, the deviation between the manual loading position and the retrieved loading position equals the modification.

If the position memory is configured to store a set of loading positions, the comparison may be performed with all loading positions and the manual loading position may be stored only if the minimum difference to a stored loading position exceeds the filling deviation threshold.

According to a further aspect, the invention is directed towards systems for ambulatory drug infusion, comprising a device as described above as well as a filling apparatus. The filling apparatus is configured to define the displacement of a cartridge plug within a cartridge body, and upon the being filled. The displacement of the plug is directly correlated with the filling volume.

The displacement of the plug is advantageously stepwise or continuously adjustable to allow different filling volumes of the cartridge. The filling apparatus may especially act as a stop which limits the displacement of the plug upon the cartridge being filled.

A filling apparatus as described here allows filling cartridges to a defined filling volume in a simple and reproducible way. Therefore, manual adjustment of the plunger position is not necessary. An embodiment for such a filling apparatus is described in more detail below in the framework of exemplary embodiments of the invention.

According to a still further aspect, the invention is directed towards methods for loading a cartridge into an ambulatory infusion device, comprising the steps of retrieving a stored loading position from a position memory of the device and controlling a drive of the device to displace a plunger of the drive into the retrieved loading position. The device may especially be a device according to the invention as described above. All computation and steps as well as the overall control of the method steps carried out by the device are controlled by the device controller where not explicitly mentioned.

The method advantageously comprises the step of entering a manual loading position via an input unit. The manual loading position may be entered by modifying a reference position or a retrieved loading position or by directly entering the manual loading position or a corresponding filling volume as described above. The method may further comprise the step of storing the manually entered loading position as stored loading position into the position memory. The method may further comprise the step of displacing the plunger along with entering a manual loading position.

The method may comprise the step of comparing the manual loading position and at least one stored loading position and of selectively storing the manual loading position in the position memory in dependence of the comparison. The comparison may be performed based on a filling deviation threshold as described above.

The method may comprise the step of storing the current plunger position when removing a cartridge as stored loading position in the position memory. This kind of embodiment allows simple re-inserting of a cartridge which is temporarily removed since it avoids manual positioning the plunger when re-inserting the previously used cartridge. The step of storing the plunger position when removing a cartridge may be performed every time a cartridge is removed or may be performed selectively. It may especially be performed only if the remaining drug volume in the cartridge exceeds a minimum storage threshold volume. For a standard insulin cartridge of 300 I.U. maximum filling volume, the minimum storage threshold volume may, for example be 50 I.U. For a remaining drug volume below the minimum storing threshold volume, re-inserting is not reasonable.

The method may comprise the step of selecting the stored loading position to be retrieved from a set of stored loading positions.

The method may comprise the step of filling the cartridge to a defined filling volume using a filling apparatus, the filling apparatus being adapted to define the displacement of a plug of the cartridge upon being filled. The filling apparatus may especially be a filling apparatus of a system for ambulatory drug infusion as defined above.

Further aspects and embodiments of the method for loading a cartridge according to the invention may be derived from embodiments of a device according to the invention as described above and from the examples described in more detail in the following.
Figure 1 shows an outside view of a device according to the present invention (discussed above).
Figure 2a schematically shows a plunger and a cartridge plug in correct engagement; Figure 2b shows a plunger and a cartridge plug in incorrect engagement (discussed above).
Figure 3 schematically shows a structural view of a device according to the present invention.
Figure 4 shows the steps of displacing the plunger into the loading position according to an exemplary embodiment of the invention.
Figure 5 shows the steps of displacing the plunger into the loading position according to a further exemplary embodiment of the invention.
Figure 6 schematically shows a filling apparatus for a drug cartridge as it may be used in a system for ambulatory drug infusion according to the present invention.

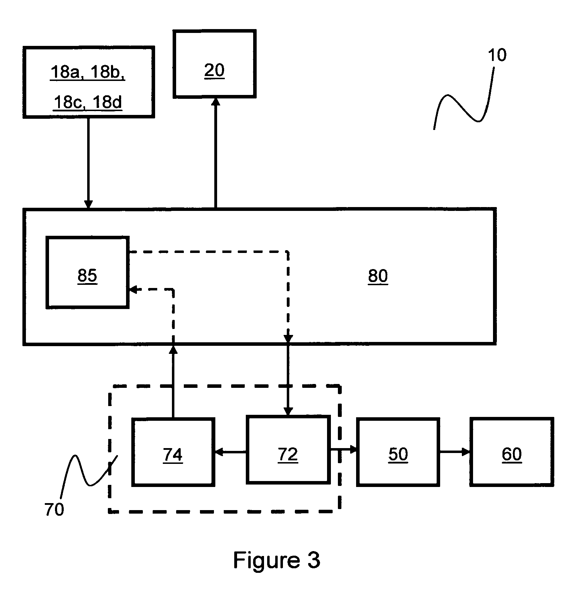

Figure 3 shows a simplified structural view an infusion device in accordance with the present invention. The design of the device may, for example, be in accordance with Figure 1 as referred to above or in accordance with any other design known in the art for such devices.

The device comprises a drive 70 which is made by a drive unit 72 and a feedback unit 74. The drive unit 72 is a spindle drive comprising an electrical motor, for example a DC motor, a stepper motor or a brushless DC motor in engagement with a plunger (not visible) which may, by actuating the motor, be displaced in or against the delivery direction *a*. The plunger is designed to act on a plug of the cartridge 50, such that drug can be forced out of the cartridge 50 and into the catheter 60 in a controlled way. The feedback unit 74 comprises a force sensor which is designed to measure the force exerted by the drive unit 72 onto the plug or the cartridge as well as a rotary encoder. The feedback unit is used for error detection as well as for detecting if the plunger assumes its fully retracted reference position.

Operation of the infusion device and in particular of the drive 70 is controlled by an electronic controller 80 which is made by state-of-the-art electronic circuitry as known for such devices. The term "controller" refers to the control circuitry as a whole and may include micro controllers, ASICS, memory, timers, supervision units, power circuitry for driving the drive unit 72, and the like.

Besides the functional components shown in Figure 3, the device 10 may comprise further components such as a power supply, acoustical and/or tactile indicators, and one or multiple communication interfaces. In addition or alternatively to the input unit 18 and/or the display 20, these elements may be comprised by a remote device and operatively coupled to the infusion device via a communication interface.

The infusion device 10 further comprises a position memory 85 which is configured to store at least one loading position of the plunger of the drive 70. The controller unit 80 may retrieve a stored loading position from the position memory 85 and control the drive 70 to move the plunger to the retrieved positions. The position memory 85 may be dedicated memory or may be integral with further memory of the device.

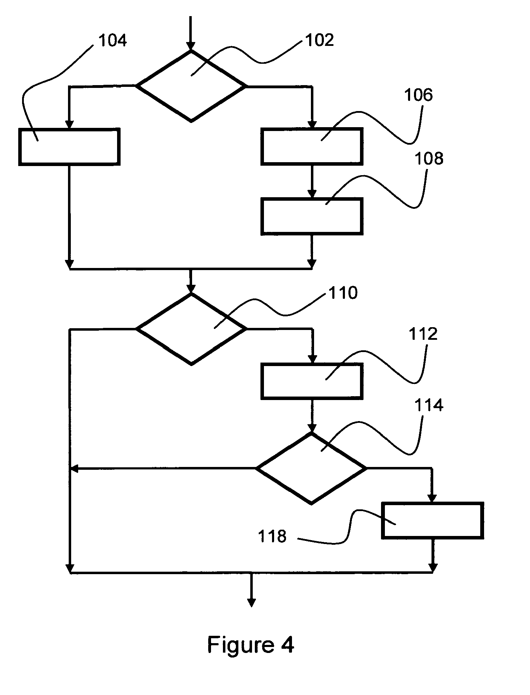

Figure 4 shows the steps of displacing the plunger into the loading position prior to loading a new cartridge into the cartridge compartment of the infusion device according to an exemplary embodiment of the invention. These steps are performed after removing a previously used cartridge and prior to inserting the new cartridge into the cartridge compartment of the device.

In step 102, the user inputs whether the plunger shall be displaced into the reference position, corresponding to a cartridge filled to its maximum volume, or into a stored loading position stored in the position memory. In dependence of the user's selection, the controller either controls the drive to displace the plunger into the reference position in step 104, or retrieves a stored loading position from the position memory in step 106 and controls the drive to displace the plunger into the retrieved loading position in step 108. The retrieved loading position was previously stored in the position memory (see following steps as described below). In step 110, the user inputs whether to directly use the position into which the plunger was displaced in step 104 or 108, respectively as loading position, or whether he wants to modify the loading position. In the first case, the process of positioning the plunger is finished. In the second case, the plunger may be displaced in distal and proximal direction using the input unit, for example the buttons 18c and 18d as shown in Figure 1 in step 112. While doing so, the corresponding filling volume is indicated by the device display. Manually modifying the loading position is necessary if the cartridge to be loaded is neither filled to its maximum filling volume nor to the filling volume corresponding to the stored loading position. Manual fine adjustment may further be required in order to compensate for variability in filling the cartridges.

After positioning the plunger in step 112, it is decided whether this new manual loading position should be stored into the position memory. Therefore, the user may be prompted to manually do the selection. Alternatively, the selection could be performed automatically by the controller, based on the filling deviation threshold as described above. Storing the modified loading position into the position memory is performed in step 118. The storing may be indicated to the user.

The steps as shown in Figure 4 may be modified in several ways. The position memory may especially be designed to store a set of multiple loading positions in a set of memory registers, each corresponding to a different filling volume of a cartridge. In this case, the step 106 of retrieving a stored loading position from the position memory advantageously comprises a step of entering from which register the loading position shall be retrieved. For this type of design, the step 118 comprises the step of selecting into which memory register of the position memory the adjusted plunger position shall be stored. This selection may be done based on a user input or performed automatically. For example, the oldest one of the stored loading positions or the stored loading position with the minimum difference to the modified loading position may be selected for replacement. The different loading positions stored in the set of memory registers are advantageously identified and indicated to the user by identifying labels, such as characters, numbers, and/or the corresponding filling volume which is advantageously displayed on the device display.

In a further variation, the administration device may be designed such that a loading position into which the plunger was manually moved in step 112 is automatically stored as loading position in the position memory and the user may command the device to use this position as loading position in step 102. For this kind of embodiment, the step 118 of storing the adjusted loading position is automatically carried out along with modifying the loading position in step 112, and step 114 may be omitted. When loading a new cartridge, this kind of embodiment allows displacing the plunger into either of the reference position, corresponding to a cartridge being filled to its maximum filling volume, or to the last previous loading position different from the reference position.

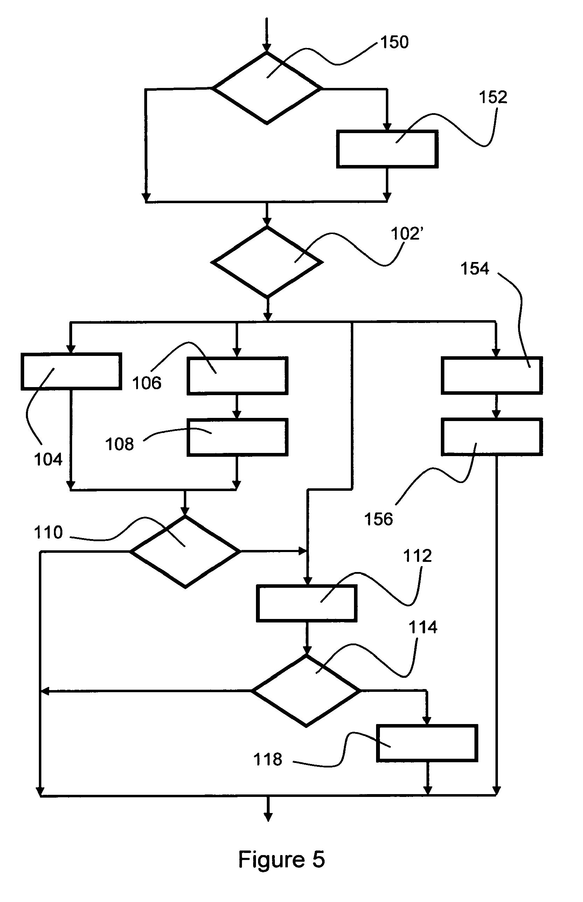

Figure 5 shows the steps of displacing the plunger into the loading position according to a further exemplary embodiment of the invention offering additional options. In the following, only those steps are discussed which are not present or are different as compared to the embodiment according to Figure 4.

In step 150, the user inputs if he wants to store the current plunger position when removing the previously used cartridge. If he decides to do so, the plunger position is stored into the position memory in step 152 as re-loading position. Storing the plunger position when removing a previously used cartridge is favorable if a cartridge is replaced which has not been fully emptied and is to be re-loaded again later on. The steps 150 and 152 are carried out immediately before or after removing the previously used cartridge. One or multiple memory registers may be provided by the position memory for that purpose. Alternatively, to an input made by the user, the selection may be done automatically taking into account a minimum storing volume threshold as described above.

The selection step 102' is similar to the step 102 as described above, but offers additional options. In the embodiment shown in Figure 5, the plunger is displaced into the reference position in step 104 or into a retrieved loading position in step 108 and may subsequently be further adjusted in step 12. If however, the loading position for a cartridge to be loaded into the device is neither of the reference position nor a stored loading position, it may be advantageous, after removing the previous cartridge, to directly proceed to the step 112 of manually entering the loading position instead of displacing the plunger into the reference position or a stored loading position first.

In addition, the selection step 102' allows selecting a re-loading position previously stored in step 152 as loading position. In this case, a re-loading position is retrieved from the position memory 154 and the controller controls the drive to displace the plunger into the retrieved re-loading position in step 156. In this case of moving the plunger to a re-loading position, further manual modification in step 112 is not necessary, such that the process of displacing the plunger terminates with step 156. Since a re-loading position typically is unique and related to a specific previously removed cartridge, the re-loading position may be deleted from the position memory after displacing the plunger into the retrieved re-loading position.

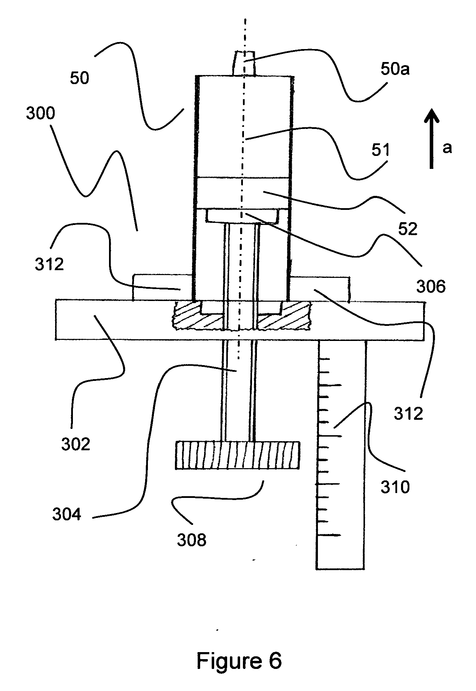

Figure 6 shows a filling apparatus as it may be used in a system for ambulatory drug infusion and/or in a method for loading a cartridge into a device according to the present invention.

The filling apparatus 300 comprises a plate-like base 302 with a fixture 312 for releasable fixing a cartridge 50 to be filled. The fixture 312 may, for example, be a clamp which radially clams the cartridge body 51 of the cartridge 50.

The filling apparatus 300 comprises a threaded spindle 304 which may project into the cartridge body 51. The base 302 has an inner thread engaging the spindle 304. At its distal end, the spindle 304 carries a limiter 306 formed by a cylindrical plate. At its proximal end, the spindle 304 carries a knurled knob 308 for rotating the spindle 304, thus adjusting the projection length of the spindle 304 into the cartridge body 51. In addition, a scale 310 is provided which is calibrated in volume, for example International Units of insulin. Even though not shown, the filling apparatus 300 advantageously additionally comprises a blocking structure for blocking the spindle 304. This allows the user to only once set the filling volume and to subsequently fill a cartridge whenever required to this filling volume in a simple and reproducible way.

For filling the cartridge 50, the plug 52 is first in its most distal position. Subsequently, drug is forced into the cartridge body 51 through the cartridge connector 50a, for example, using a syringe or any other over-pressure device. The drug in the cartridge body 51 displaces the plug 52 against the delivery direction *a*, until it contacts the stopper 306, thus limiting the filling volume. The relation between the displacement distance of the plunger 90 and the filling volume of the cartridge 50 is given by the cartridge cross-section.

It will be appreciated by a person skilled in the art that Figure 6 mainly illustrates the general principle design of a filling apparatus to be used in a system for ambulatory drug infusion according to the present invention while a variety of detailed designs of the filling apparatus may be used. For example, the scale 310 may be of different design and have the design of a micrometer scale, a gauge, or the like. Instead of the knob 308, an electrical drive may be foreseen. A rotational encoder and/or other sensor elements may be provided on the spindle 304 in order to automatically determine the spindle position and thus the maximum filling volume of the cartridge. The maximum filling volume may be transferred to an ambulatory infusion device in accordance with the invention, thus fully avoiding the manual entry of loading positions.

An alternative filling apparatus may engage the plug by a threaded engagement or the like and pull the plug 52 against the delivery direction *a* in a defined way, thus sucking drug into the cartridge 50. For those embodiments, the ambulatory infusion device itself may additionally serve as filling apparatus.

### Reference signs

- 10: ambulatory infusion device
- 12: housing
- 13: cartridge compartment bottom surface
- 14: cartridge compartment
- 18: input unit
- 20: display
- 50: cartridge
- 50a: cartridge connector
- 51: cartridge body
- 52: plug
- 54: proximal front surface of the plug
- 55: cavity
- 60: catheter
- 62: catheter connector
- 65: adapter
- 70: drive
- 72: drive unit
- 74: feedback unit
- 80: controller
- 85: position memory
- 90: plunger
- 91: connector plate
- 92: threaded bolt
- 93: distal front surface of connector plate
- 94: proximal front surface of connector plate
- 95: housing face
- 300: filling apparatus
- 302: base
- 304: spindle
- 306: limiter
- 308: knob
- 310: scale
- 312: fixture

## Claims

1. Ambulatory infusion device for the infusion of a liquid drug into a patient's body over an extended time period, comprising:
a) a housing (12) having a cartridge compartment (14), the cartridge compartment (14) being designed to receive a cartridge (50);
b) a drive (70) having a plunger (90), the plunger (90) being adapted to engage a plug (52) of the cartridge (50), the drive (70) being configured to displace, via the plunger (90), the plug (52) within cartridge body (51) in a controlled way;
c) an electronic controller (80),configured to control operation of the drive (70),
**characterized in that** the infusion device (10) further comprises
d) a position memory (85), configured to store at least one loading position of the plunger (90), and **in that**
e) the controller (80) is configured to retrieve a stored loading position from the position memory (85) and to control the drive (70) to displace the plunger (90) into the retrieved loading position.

2. Ambulatory infusion device (10) according to Claim 1, **characterized in that** the plunger (90) couples the plug (52) by releasable threaded engagement.

3. Ambulatory infusion device (10) according to either of the preceding claims,
**characterized in that** the device (10) is configured for entering a manual loading position via an input unit (18).

4. Ambulatory infusion device (10) according to either of the preceding claims,
**characterized in that** the position memory (85) comprises a set of memory registers, each memory register being configured to store a stored loading position and **in that** the controller (80) is configured to retrieve a stored loading position from the set of stored loading positions and to control the drive (70) to displace the plunger (90) into the retrieved loading position.

5. Ambulatory infusion device (10) according to either of the preceding claims,
**characterized in that** the controller (80) is configured to modify a retrieved loading position based on an input entered via an input unit (18) and to control the drive to displace the plunger (90) into the modified loading position.

6. Ambulatory infusion device according to Claim 5, **characterized in that** the device (10) is configured to compare the manual loading position and a stored loading position and to selectively replace the stored loading position in the position memory (85) by the manual loading position in dependence of the comparison result.

7. System for ambulatory drug infusion, comprising:
a) an ambulatory infusion device (10) according to either of Claim 1 to Claim 6;
b) a filling apparatus, adapted to define the displacement of a cartridge plug (52) within a cartridge body (51) upon being filled, thus defining the filling volume of the cartridge (50).

8. Method for loading a cartridge (50) into an ambulatory infusion device (10), comprising the steps of retrieving a stored loading position from a position memory (85) of the device (10) and controlling a drive (70) of the ambulatory infusion device (10) to displace a plunger (90) of the drive (70) into the retrieved loading position.

9. Method according to Claim 8, **characterized in that** the method comprises the step of entering a manual loading position via an input unit (18).

10. Method according to Claim 9, **characterized in that** the method comprises the step of storing the manual loading position as stored loading position into the position memory (85).

11. Method according to Claim 9 or Claim 10, **characterized in that** the method comprises the step of displacing the plunger (90) while entering the manual loading position.

12. Method according to either of Claim 9 to Claim 11, **characterized in that** the method comprises the step of comparing the manual loading position and at least one stored loading position and to selectively store the manual loading position in the position memory (85) in dependence of the comparison.

13. Method according to either of Claim 8 to Claim 10, **characterized in that** the method comprises the step of storing the current plunger position when removing a cartridge as stored loading position in the position memory (85).

14. Method according to either of Claim 8 to Claim 12, **characterized in that** the method comprises the step of selecting the stored loading position to be retrieved from a set of stored loading positions.

15. Method according to either of Claim 9 to Claim 14, **characterized in that** the method comprises the step of filling the cartridge (50) to a defined filling volume using a filling apparatus (300), the filling apparatus being adapted to define the displacement of a plug (52) of the cartridge (50) upon being filled within the cartridge body (51).
